# EUROPEAN PATENT SPECIFICATION

(11) **EP 0 427 823 B1**
(45) Date of publication and mention of the grant of the patent: **18.01.1995**
(21) Application number: 90906627.6
(22) Date of filing: 10.04.1990
(51) Int. Cl.: G01N 1/02, G01N 1/28, G01N 33/72

(54) **SPECIMEN COLLECTION DEVICE AND METHOD**
SPECIMEN-SAMMELANORDNUNG UND VERFAHREN
DISPOSITIF ET PROCEDE DE PRELEVEMENT DE SPECIMENS

(30) Priority: 09.05.1989 US 349274
(43) Date of publication of application: 22.05.1991
(73) Proprietor: BECKMAN INSTRUMENTS, INC., Fullerton, CA 92634 (US)
(72) Inventor: BAKER, Josefina, T., Cupertino, CA 95014 (US); MERVINE, Robert, W., Drexel Hill, PA 19026 (US); HUCKO, Josephine, T., San Jose, CA 95128 (US)
(74) Representative: Ede, Eric
(86) International application number: US9001962
(87) International publication number: WO9013802

(56) References cited:
- EP-A- 0 239 265

## Description

This application is related to co-pending patent application entitled "Device and Method for Collecting Fecal Occult Blood Specimens", serial number EP-A-0428643.

### Field of the Invention

The present invention relates generally to the field of specimen collection and more specifically to devices and methods useful in collecting human fecal specimens for use in fecal occult blood testing.

### Background of the Invention

Fecal occult blood testing has become a popular, widely used procedure to detect relatively small amounts of blood in fecal specimens. This wide use and popularity arises primarily because fecal occult blood testing is non-invasive, simple and inexpensive to perform. -Because the presence of fecal occult blood in a specimen is a symptom that may be associated with colon cancer or a precursor to colon cancer, fecal occult blood testing is often routinely used on a screening basis. The routine screening of patients using fecal occult blood testing has helped to detect colon cancer at a stage where the disease is readily treatable.

A popular form of fecal occult blood testing utilizes a guaiac treated test sheet upon which a specimen of fecal material is smeared. A developing solution is applied to the opposite side of the sheet, yielding a blue color suggesting blood may be present in the fecal specimen. As the need for more specific fecal occult blood tests has been recognized, the use of immunochemical testing techniques has gained popularity.

Regardless of the technology used in performing the fecal occult blood test, there has been an on-going need to obtain, transport and process those specimens in a manner that is as convenient and aesthetically acceptable as possible and such that the specimen is not degraded. One form of specimen collection device that has gained wide popularity is a slide formed from folded paper or cardboard. The slide includes guaiac treated paper to which the fecal specimen is applied and a cover which is closed once the specimen application is completed. A flap in the back of the slide may be opened to reveal the back of the guaiac treated paper for subsequent application of developer and observation of the paper to determine the presence of the blue color. Examples of such a test slide are disclosed in U.S. Patents 3,996,006 and 4,365,970.

Similar approaches have been utilized in collecting specimens for use in immunochemical tests. Typically, such tests require that a substrate such as paper to which the fecal specimen has been applied must be deposited in a vial or microtiter plate. One example of collection device is a specimen slide distributed by Fujirebio, Inc. which includes a sheet of filter paper onto which the fecal specimen is applied. The cover of the slide is closed and the slide is sent to a laboratory for analysis. To remove specimen from the device for analysis, the cover of the slide is again opened, a portion of the slide carrying the filter paper is pulled away, and a pre-punched circle is removed from the filter paper for analysis. Unfortunately, the front of the Fujirebio slide must be re-opened by the medical technologist and the technologist must grasp an area inside the slide immediately adjacent the fecal smear, thus unnecessarily exposing the medical technologist to the specimen.

Other examples of sampling devices and methods for immunological tests are disclosed in U.S. Patents 4,645,743 and 4,789,629. These devices, however, include a separate insert to which the fecal specimen is applied by the patient. The insert is removed from the device and the insert is then punched or sectioned to obtain a portion of the insert suitable for immunological analysis. The use of such a removable insert presents a disposal problem in addition to the device itself. Also, because the insert must be punched or sectioned, additional tools must be cleaned after each use, further complicating the process and adding expense.

### Summary of the Invention

The present invention overcomes the limitations found in the prior art. A fecal occult blood specimen collection device in accordance with the present invention includes front and back panels, an aperture in the front panel, and a cover. A sheet which is adapted for receiving the fecal specimen is positioned between the front and back panels and includes a plurality of perforations defining removable portions which appear through the aperture. Once a fecal specimen has been applied through the aperture to the sheet, the cover is closed over the aperture. A flap in the rear panel may be opened for convenient removal of one or more of the removable portions without having to open the cover.

Thus, the specimen collection device of the present invention, as well as the method of the present invention, provide a simple and neat means for obtaining and transporting specimens and convenient handling of the specimens for testing purposes. The device may be used for collecting other types of specimens, such as blood from finger pricks or material collected using swabs, and may be used for testing analytes other than blood in feces and other specimens.

### Brief Description of the Drawings

Figure 1 is a perspective view the front panel and cover of a specimen collection device in accordance with the present invention.

Figure 2 is a perspective view of the back panel of the device of Figure 1.

Figure 3 is a perspective view of another embodiment of the device of Figure 1.

Figure 4 is a perspective view of the back panel of the device shown in Figure 3.

### Detailed Description

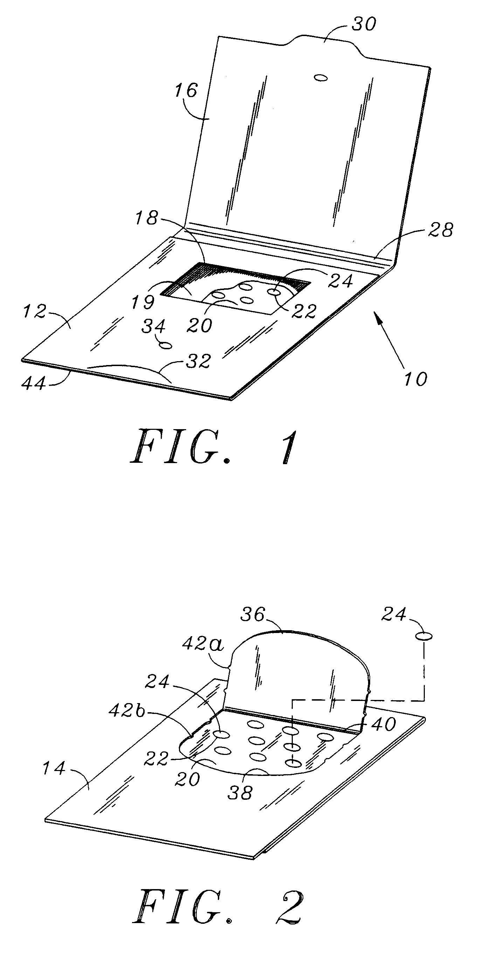

With reference to Figures 1 and 2, a device in accordance with the present invention is in the form of a specimen slide 10 and includes a front panel 12, back panel 14, and a cover 16. A rectangular aperture 18 is formed in the front panel. A thin sheet of mesh or porous screening material 19 (shown partially cut-away in Figure 1) and a specimen sheet 20 are retained between the front panel 12 and the back panel 14. The screening material 19 is a high strength, high porosity tissue composed, for example, of cellulosic fiber or synthetic materials, such as polyester or nylon mesh. Suitable materials include "Hollytex" brand material, grade 3257, from Eaton-Dikeman Division of Filtration Sciences Corporation, Mount Holly Springs, Pennsylvania, and grade 785 tissue from the C. H. Dexter Division of The Dexter Corporation, Windsor Locks, CT. The screening material 19 overlies the specimen sheet 20 and is disposed between the aperture 18 and specimen sheet 20. The specimen sheet 20 is formed, for example, from filter paper and includes a plurality of perforations 22 which define circular removeable portions 24 of the specimen sheet 20 that can be easily removed as is described hereinbelow. The cover 16 includes a tab 30 formed at the outer edge of the cover 16 and is adapted to fold along a hinge line 28. The tab 30 is adapted to engage a semi-circular cut 32 formed in the front panel 12 to thus close the specimen slide 10 once a specimen has been applied through the aperture 18 and screening material 19 to the specimen sheet 20.

A flap 36 (shown in its opened position in Figure 2) is formed in the back panel 14 by an outline of perforations 38 and a crease 40 defining a hinge. The perforations 38 are spaced to define a plurality of bridges 42, each comprising bridge portions 42a, 42b, between the flap 36 and the surrounding portion of the back panel 14. The bridges 42 hold the flap 36 in place until the bridges 42 are broken as the flap 36 is opened along the crease 40. When opened as illustrated in Figure 2, the flap reveals the back of the specimen sheet 20 and the removable portions 24.

Preferably, the specimen slide 10 is assembled using a single length of cardboard or paper into which the aperture 18, cut 32 and perforations 38 are die-cut. The specimen sheet 20, already including the perforations 22, and the screening material 19 are positioned against the inside of the back panel 14. A bend is formed at edge 44 and the front and back panels 12, 14 are fixed together by means of a suitable adhesive or glue. The cover 16 is folded along the hinge line 28 and removably affixed to the front cover by means of the drop or dot of glue 34.

In use, a patient opens the cover 16 breaking the cover 16 away from the glue 34, revealing the aperture 18. A specimen of fecal material or other specimen is smeared with a suitable applicator through the screening material 19 and onto the specimen sheet 20. The cover 16 is closed with the tab 30 beneath the cut 32 and the specimen slide 10 is returned to the physician's office or laboratory for analysis.

To remove a portion of the specimen from the specimen slide 10, the flap 36 is freed from the back panel 14 by breaking the bridges 42 and is opened. Using a suitable implement such as tweezers, one or more of the removable portions 24 to which a portion of the specimen was applied is easily separated from the specimen sheet 20 and may be deposited in a microtiter plate well or other suitable test vessel for subsequent immunochemical assay.

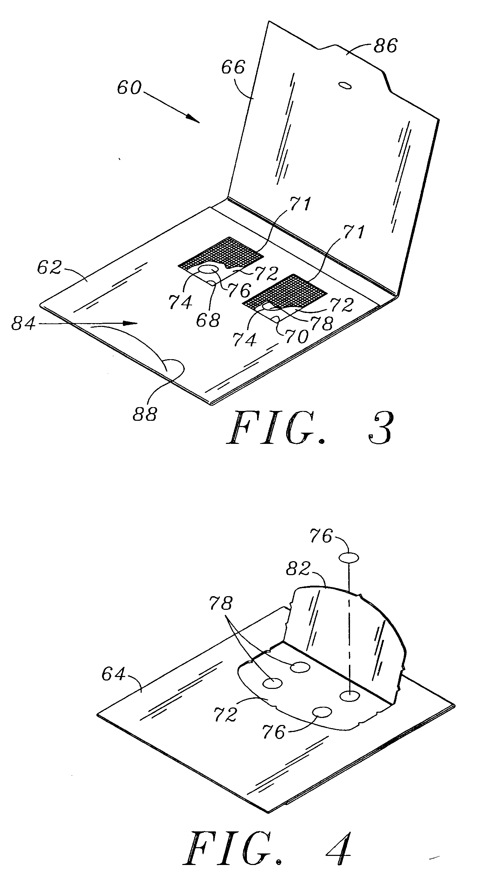

With reference now to Figures 3 and 4, another embodiment of a specimen slide 60 is illustrated. The specimen slide 60 includes front panel 62, rear panel 64 and cover 66. Two apertures 68 and 70 are formed through the front panel 62. A thin sheet of screening material 71 and a specimen sheet 72 are retained between the front panel 62 and rear panel 64. The screening material 71 overlies the specimen sheet 72 and is disposed between the apertures 68 and 70 and the specimen sheet 72. The specimen sheet 72 includes a plurality of perforations 74 which define two removable portions 76 aligned with and visible through the aperture 68 and two removable portions 78 aligned with and visible through the aperture 70. As an alternative, the plurality of perforations 74 may be arranged in a more densely packed pattern such as illustrated in Figure 2. With such an alternative pattern, the specimen sheet,72 may be retained between the front and rear panels 62 and 64 without the need to carefully align particular perforations with the apertures 68 and 70 as is necessary with the pattern illustrated in Figure 4.

A flap 82 is formed in the back panel 64. The flap 82 is similar to the flap 36 of the slide 10 and may be opened to reveal the removable portions 76 and 78. The specimen side 60 may be assembled in a fashion similar to that described for the specimen slide 10 with the cover 66 initially held in place by means of a glue dot 84.

The use of the specimen slide 60 is similar to that of the specimen slide 10. The cover 66 is separated from the glue dot 84 and is opened to reveal the apertures 68, 70. However, two specimens from different sites of the fecal material may be applied through the two apertures to 68 and 70. The cover 66 is then closed and is secured to the front panel 62 by means of a tab 86 and slit 88. To remove a specimen from the slide 60, the flap 82 is opened and one or more of the removable portions 76 or 78 carrying a portion of the respective specimens are removed and used for analysis of the specimen.

Various modifications to the present invention will be readily apparent to those skilled in the art. For example, the specimen slide 10 or 60 may be constructed without the screening material 19 or 71. The shapes and sizes of the apertures 18, 68 and 70 may vary according to, for example, the size of the specimen slide or the amount of specimen that is to be applied to the specimen sheet. For example, a smaller aperture may have the effect of concentrating the specimen in a smaller area, improving the reproducibility of the specimen gathering technique. Also, the sizes of the removeable portions 24, 76 and 78 may be varied to carry more or less specimen to thereby accommodate differing sensitivities of testing methodologies.

The specimen slides 10 and 60 advantageously allow access to the fecal specimens without reopening the portion of the slides to which the specimens were originally applied. Also, the use of removable portions of the specimen sheets for subsequent analysis is neat and does not produce additional sub-parts or component which may require separate disposal. Both of the specimen slides 10 and 60 provide a convenient and aesthetically improved means for collecting and handling fecal specimens for immunochemical analysis.

The present invention is not to be limited to the detailed description contained herein but is to be afforded the full scope of the appended claims and all equivalents thereto.

## Claims

1. A specimen slide (10, 60) comprising:
a front panel (12,62);
back panel (14, 64);
the front including an aperture (18, 68);
a sheet (20, 72) carried between the front and back panels and positioned for receiving a fecal specimen smear through the aperture;
a cover (16, 66) adapted to overlie and close the front panel aperture; and flap means (36, 82) in the rear panel opposite said sheet;
characterized in that the sheet includes a plurality of perforations (22, 74) defining removable portions (24) of the sheet appearing through the aperture and said flap means provide access to the removable portions.

2. A slide as in claim 1 wherein the slide further includes screen means (19, 71) between the aperture and the sheet for blocking excessive specimen from being applied to the sheet.

3. The specimen slide as in claim 2 wherein the slide further includes a second aperture (70) in the front panel, the sheet means includes a further plurality of perforations defining second removable portions (76) appearing through the second aperture, and the flap means is opposite the first and second removable portions.

4. A method for obtaining a specimen suitable for use in testing, using a specimen slide (10, 60) including a front panel (12, 62) having an aperture (18, 68) and a back panel (14, 64) having an openable flap (36, 82), a specimen receiving sheet (20, 72) between the front and back panels, and a cover (16, 66) hinged to cover the aperture, the specimen slide characterized in that the sheet includes a plurality of perforations (22, 74) defining a removable portion (24) of the sheet appearing through the aperture, the method comprising:
(a) obtaining a specimen;
(b) smearing at least a portion of the specimen onto the receiving sheet through the aperture;
(c) closing the cover over the aperture;
(d) opening the flap; and
(e) removing the removable portion of the specimen receiving sheet via the open flap.

5. A method as in claim 4 wherein the slide includes screen means (19, 71) between the aperture and the specimen receiving sheet and the step of smearing at least a portion of the specimen further includes applying the specimen through the screen means onto the specimen receiving sheet.

6. A method as in claim 4 wherein the step of obtaining a specimen includes obtaining a fecal specimen.

## Patentansprüche

1. Proben-Gleitträger (10,60) umfassend:
eine Vorderwandung (12;62);
eine Rückwandung (14,64);
wobei die Vorderwandung eine Öffnung (18,68) aufweist;
ein zwischen der Vorder- und der Rückwandung getragenes Blatt (20,72) in solcher Anordnung, daß es durch die Öffnung hindurch einen Streichauftrag einer Stuhlprobe aufnehmen kann;
einen Deckel (16,66) in solcher Ausbildung, daß er über der Vorderwandungs-Öffnung liegt und diese verschließt;
sowie Klappenmittel (36,82) in der Rückwandung in Ausrichtung mit dem genannten Blatt;
dadurch **gekennzeichnet**, daß das Blatt mehrere Perforationen (22,74) aufweist, welche zur Entnahme abtrennbare Bereiche (24) des durch die Öffnung erscheinenden Blatts definieren, und daß die genannten Klappenmittel Zugang zu den abtrennbaren Bereichen bilden.

2. Probenträger nach Anspruch 1, bei welchem der Träger des weiteren Masken- bzw. Siebmittel (19,71) zwischen der Öffnung und dem Blatt aufweist, zur Verhinderung eines übermäßigen Probenauftrags auf dem Blatt.

3. Probenträger nach Anspruch 2, bei welchem der Träger des weiteren eine zweite Öffnung (70) in der Vorderwandung aufweist, die Blattmittel eine weitere Mehrzahl von Perforationen aufweisen, welche durch die zweite öffnung erscheinende zweite zur Entnahme abtrennbare Bereiche (76) definieren, und daß die Klappenmittel mit den ersten und zweiten abtrennbaren Bereichen ausgerichtet sind.

4. Verfahren zur Gewinnung einer zur Verwendung in Tests geeigneten Probe, unter Verwendung eines Probengleitträgers (10,60), der eine Vorderwandung (12,62) mit einer Öffnung (18,68) und eine Rückwandung (14,64) mit einer zu öffnenden Klappe (36,82), ein Probenaufnahmeblatt (20,72) zwischen der Vorder- und der Rückwandung, und einen Schwenk-Deckel (16,66) zur Abdeckung der Öffnung aufweist, wobei der Probenträger dadurch gekennzeichnet ist, daß das Blatt mehrere Perforationen (22,74) aufweist, welche einen zur Entnahme abtrennbaren Bereich (24) des durch die Öffnung erscheinenden Blatts definieren, wobei das Verfahren die Schritte umfaßt:
(a) Gewinnen einer Probe;
(b) Bestreichen des Aufnahmeblatts durch die öffnung hindurch mit wenigstens einem Teil der Probe;
(c) Schließen des Deckels über der Öffnung;
(d) Öffnen der Klappe; und
(e) Entnahme des abtrennbaren Bereichs des Proben-Aufnahmeblatts durch die geöffnete Klappe.

5. Verfahren nach Anspruch 4, bei welchem der Träger Masken- bzw. Sieb-Mittel (19,71) zwischen der Öffnung und dem Probenaufnahmeblatt aufweist und der Verfahrensschritt des Auftragens wenigstens eines Teils der Probe des weiteren den Auftrag der Probe durch die Masken- bzw. Siebmittel hindurch auf das Proben-Aufnahmeblatt umfaßt.

6. Verfahren nach Anspruch 4, bei welchem der Schritt der Entnahme bzw. Gewinnung einer Probe die Entnahme bzw. Gewinnung einer Stuhlprobe umfaßt.

## Revendications

1. Lame pour prélèvement (10, 60) comprenant :
un panneau avant (12, 62);
un panneau arrière (14, 64);
l'avant comprenant une ouverture (18, 68);
une feuille (20, 72) portée entre les panneaux avant et arrière et Positionnés pour recevoir un frottis de prélèvement fécal à travers l'ouverture;
un couvercle (16, 66) conçu pour reposer sur et fermer l'ouverture du panneau avant; et un moyen formant rabat (36, 82) dans le panneau arrière opposé à ladite feuille; caractérisée en ce que la feuille comprend une pluralité de perforations (22, 74) définissant des portions amovibles (24) de la feuille apparaissant à travers l'ouverture, et ledit moyen de rabat permettant l'accès aux portions amovibles.

2. Lame selon la revendication 1, dans laquelle la lame comprend en outre un moyen formant écran (19, 71) entre l'ouverture et la feuille pour empêcher que du prélèvement excédentaire soit appliqué à la feuille.

3. Lame pour prélèvement selon la revendication 2, dans laquelle la lame comprend en outre une deuxième ouverture (70) dans le panneau avant, le moyen formant feuille comprenant une pluralité supplémentaire de perforations définissant des deuxièmes portions amovibles (76) apparaissant à travers la deuxième ouverture, et le moyen formant rabat est opposé auxdites première et deuxième portions amovibles.

4. Procédé pour l'obtention d'un prélèvement approprié pour l'utilisation pour un test, en utilisant une lame pour prélèvement (10, 60) comprenant un panneau avant (12, 62) possédant une ouverture (18, 68) et un panneau arrière (14, 64) possédant un rabat ouvrant (36, 82), une feuille de réception de prélèvement (20, 72) entre les panneaux avant et arrière et un couvercle (16, 66) articulé pour couvrir l'ouverture, la lame pour prélèvement étant caractérisée en ce que la feuille comprend une pluralité de perforations (22, 74) définissant une portion amovible (24) de la feuille apparaissant à travers l'ouverture, le procédé comprenant les étapes consistant à :
(a) obtenir un prélèvement;
(b) étaler au moins une partie du prélèvement sur la feuille de réception à travers l'ouverture;
(c) fermer le couvercle sur l'ouverture;
(d) ouvrir le rabat; et
(e) retirer la partie pouvant être retirée de la feuille de réception de prélèvement via le rabat ouvert.

5. Procédé selon la revendication 4, dans lequel la lame comprend un moyen formant écran (19, 71) entre l'ouverture et la feuille de réception de prélèvement, et l'étape consistant à étaler au moins une partie du prélèvement comprend en outre l'application du prélèvement à travers le moyen formant écran sur la feuille de réception de prélèvement.

6. Procédé selon la revendication 4, dans lequel l'étape consistant à obtenir un prélèvement comprend l'obtention d'un prélèvement fécal.
